(19) Europäisches Patentamt
European Patent Office
Office européen des brevets

(11) Publication number: **0 418 430 A1**

# (12) EUROPEAN PATENT APPLICATION

(21) Application number: **89202383.9**

(22) Date of filing: **22.09.89**

(51) Int. Cl.5: **C07D 317/64**, C07C 217/58, A61K 31/36

(43) Date of publication of application: **27.03.91 Bulletin 91/13**

(84) Designated Contracting States: **NL**

(71) Applicant: **DUPHAR INTERNATIONAL RESEARCH B.V**
**C.J. van Houtenlaan 36**
**NL-1381 CP Weesp(NL)**

(72) Inventor: **Witiak, Donald T.**
**c/o OCTROOIBUREAU ZOAN B.V.**
**P.O. Box 140, 1380 AC Weesp(NL)**
Inventor: **van Wijngaarden, Ineke**
**c/o OCTROOIBUREAU ZOAN B.V.**
**P.O. Box 140, 1380 AC WEESP(NL)**
Inventor: **Nair, Raghunathan V.**
**c/o OCTROOIBUREAU ZOAN B.V.**
**P.O. Box 140, 1380 AC WEESP(NL)**
Inventor: **Lange, Josephus H.M.**
**c/o OCTROOIBUREAU ZOAN B.V.**
**P.O. Box 140, 1380 AC WEESP(NL)**
Inventor: **den Hartog, Jacobus A.J.**
**c/o OCTROOIBUREAU ZOAN B.V.**
**P.O. Box 140, 1380 AC WEESP(NL)**

(74) Representative: **Muis, Maarten et al**
**OCTROOIBUREAU ZOAN B.V. P.O. Box 140**
**NL-1380 AC Weesp(NL)**

## (54) Phenylalkyl amine derivatives having anti-ischaemic activity.

(57) The invention relates to composition having anti-ischamemic activity which comprise as an active component at least one compound of the formula

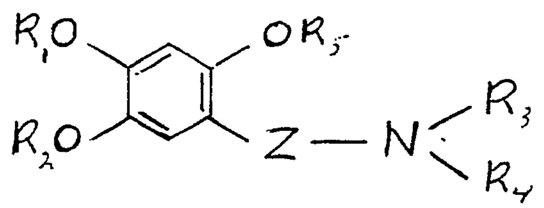

(1)

wherein:
-$R_1$ and $R_2$ independent of each other represent hydrogen, alkyl having 1-3 C-atoms, on the understanding that $R_1$ and $R_2$ are not both hydrogen, or wherein $R_1 + R_2$ together form an alkylene group having 1-3 C-atoms which may be substituted with one or more alkyl groups having 1-3 C-atoms;
-Z is methylene optionally substituted with one alkyl group having 1-3 C-atoms, or Z is ethylene of which one or both carbon atoms may be substituted with one alkyl group having 1-3 C-atoms;
-$R_3$ and $R_4$ independent of each other represent hydrogen, alkyl having 1-10 C-atoms, alkenyl or alkynyl having 3-10 C-atoms, cycloalkyl having 3-8 C-atoms, cycloalkyl-alkyl having 3-8 ring atoms and 1-5 C-atoms in the alkyl group, phenylalkyl or heteroaryl-alkyl having 1-5 C-atoms in the alkyl group, phenylalkenyl, heterayl-alkenyl, phenylalkynyl or heteroaryl-alkynyl group having 3-5 C-atoms in the alkenyl group or alkynyl group, which groups $R_3$ and R4 may be substituted with a group $(R_6)_p$ wherein $R_6$ is halogen, hydroxy, alkyl or hydroxyalkyl having 1-5 C-atoms, alkoxy having 1-3 C-atoms, S-alkyl, S(O)-alkyl or $S(O)_2$-alkyl having 1-3 C-atoms, amino, mono- or dialkylamino having 1-3 C-atoms per alkyl group, trifluoromethyl, trifluoromethoxy or cyano, and p has

EP 0 418 430 A1

the value 0-3, on the understanding that $R_3$ and $R_4$ are not both hydrogen, or wherein $R_3 + R_4$ together with the nitrogen atom form a saturated or unsaturated heterocyclic group of 5 or 6 ringatoms, which may contain a second hetero-atom from the group consisting of oxygen, sulphur and nitrogen, which ring may be substituted with phenylalkyl having 1-3 C-atoms in the alkyl group, or with a group $(R_6)_p$ wherein $R_6$ and p have the above-mentioned meanings, or which ring may be annelated with a phenyl group;

-$R_5$ is hydrogen, alkyl having 1-12 C-atoms, alkenyl or alkynyl having 3-12 C-atoms, cycloalkyl having 3-8 C-atoms, cycloalkyl-alkyl having 3-8 ring atoms and 1-5 C-atoms in the alkyl group, phenylalkyl or heteroaryl-alkyl having 1-5 C-atoms in the alkyl sub-group, phenylalkenyl, heteroaryl-alkenyl, phenylalkynyl or heteroaryl-alkynyl having 3-5 C-atoms in the alkenyl sub-group or alkynyl sub-group, which groups may be substituted with a group $(R_6)_p$, wherein $R_6$ and p have the above-mentioned meaning, and which alkyl sub-groups, alkenyl sub-groups and alkynyl sub-groups may contain a group -O-, -S- or CO, or a pharmacologically acceptable salt thereof.

# PHENYLALKYL AMINE DERIVATIVES HAVING ANTI-ISCHAEMIC ACTIVITY

The invention relates to a group of new phenylalkyl amine derivatives having interesting anti-ischaemic activity, to a method of preparing said compounds, and to pharmaceutical compositions comprising at least one of these compounds as the active component.

There is an increasing clinical interest in an effective pharmalogical symptomatic treatment for cerebral and peripheral ischaemic diseases. In patients suffering from these diseases the impaired blood supply causes an inadequate delivery of oxygen and other nutrients to the tissue as well as a diminished removal of metabolic waste products resulting in structural injury and functional deterioration.

The object of the present invention is to provide active compounds with anti-ischaemic properties.

It has been found surprisingly that compounds of formula 1

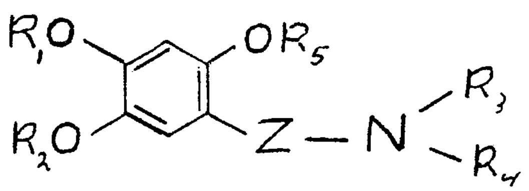

(1)

wherein

-$R_1$ and $R_2$ independent of each other represent hydrogen, alkyl having 1-3 C-atoms, on the understanding that $R_1$ and $R_2$ are not both hydrogen, or wherein $R_1 + R_2$ together form an alkylene group having 1-3 C-atoms which may be substituted with one or more alkyl groups having 1-3 C-atoms;

-Z is methylene optionally substituted with one alkyl group having 1-3 C-atoms, or Z is ethylene of which one or both carbon atoms may be substituted with one alkyl group having 1-3 C-atoms;

-$R_3$ and $R_4$ independent of each other represent hydrogen, alkyl having 1-10 C-atoms, alkenyl or alkynyl having 3-10 C-atoms, cycloalkyl having 3-8 C-atoms, cycloalkyl-alkyl having 3-8 ring atoms and 1-5 C-atoms in the alkyl group, phenylalkyl or heteroaryl-alkyl having 1-5 C-atoms in the alkyl group, phenylalkenyl, heteroaryl-alkenyl, phenylalkynyl or heteroaryl-alkynyl group having 3-5 C-atoms in the alkenyl group or alkynyl group, which groups $R_3$ and $R_4$ may be substituted with a group $(R_6)_p$ wherein $R_6$ is halogen, hydroxy, alkyl or hydroxyalkyl having 1-5 C-atoms, alkoxy having 1-3 C-atoms, S-alkyl, S(O)-alkyl or $S(O)_2$-alkyl having 1-3 C-atoms, amino, mono- or dialkylamino having 1-3 C-atoms per alkyl group, trifluoromethyl, trifluoromethoxy or cyano, and p has the value 0-3, on the understanding that $R_3$ and $R_4$ are not both hydrogen, or wherein $R_3 + R_4$ together with the nitrogen atom form a saturated or unsaturated heterocyclic group of 5 or 6 ring atoms, which may contain a second hetero-atom from the group consisting of oxygen, sulphur and nitrogen, which ring may be substituted with phenylalkyl having 1-3 C-atoms in the alkyl group, or with a group $(R_6)_p$ wherein $R_6$ and p have the above-mentioned meanings, or which ring may be annelated with a phenyl group;

-$R_5$ is hydrogen, alkyl having 1-12 C-atoms, alkenyl or alkynyl having 3-12 C-atoms, cycloalkyl having 3-8 C-atoms, cycloalkyl-alkyl having 3-8 ring atoms and 1-5 C-atoms in the alkyl group, phenylalkyl or heteroa ryl-alkyl having 1-5 C-atoms in the alkyl sub-group, phenylalkenyl, heteroaryl-alkenyl, phenylalkynyl or heteroaryl-alkynyl having 3-5 C-atoms in the alkenyl sub-group or alkynyl sub-group, which groups may be substituted with a group $(R_6)_p$, wherein $R_6$ and p have the above-mentioned meaning, and which alkyl sub-groups, alkenyl sub-groups and alkynyl sub-groups may contain a group -O-, -S- or CO,

prodrugs and pharmaceutically acceptable acid addition salts thereof have interesting and valuable anti-ischaemic properties.

Prodrugs are derivatives of these compounds which as such are inactive, from which, after splitting off an easily removable group, for example an ester group or an ether group, an active compound of formula 1 is obtained. Suitable acids with which suitable addition salts can be formed are, for example, hydrochloric acid, sulphuric acid, phosphoric acid, nitric acid, and organic acids like citric acid, fumaric acid, maleic acid, tartaric acid, acetic acid, benzoic acid, p-toluene sulphonic acid, methane sulphonic acid, etc.

One or more centres of chirality may be present in the compounds having formula 1. The invention relates both to racemates and the individual enantiomers of the compounds having formula 1.

The anti-ischaemic activity of the compounds has been determined by means of the in vivo hypobaric hypoxia test and the in vitro cardiomyocytes test. These tests were used to characterize substances with

cerebro- and/or peripheral-protective activity.

1) Hypobaric activity in vivo

Cerebro-protective activity was determined by measuring the prolongation of the survival time of conscious mice under hypobaric conditions.
Groups of 3 overnight fasted male NMRI mice (15-20 g) are dosed ip (30 mg/kg), 30 minutes before being placed in a chamber at hypobaric pressure of 200 mBar. The prolongation of the survival time is expressed in percentage increase in respiration time, compared to that of the placebo treated control group.

2) Cardiomyocytes in vitro:

Cyto-protective properties were determined in an in vitro model using isolated calcium tolerant cardiomyocytes according to L. Verdonk et al (Life Sciences, vol.38, (1986) 765-772).
Cardiomyocytes were isolated from male Wistar rat hearts. Rod shaped cells were incubated with the compound to be tested for 30 min. Injury was induced by e.g. veratrine (100µg/ml) or by hypoxia upon which the cells became rounded unless protected by the compound. After 20 min. the remaining rod-shaped cells were counted and the protecting efficacy of the compound was determined.

The compounds having formula 1, wherein the symbols have the above-mentioned meaning are new compounds, with the exception of the compound wherein $R_1 + R_2$ form a methylene group, $R_3 = R_4 = CH_3$, $R_5 = H$ and Z is the methylene group (Tetrahedron 29 , (1973), 1931). The new compounds can be prepared according to methods known per se .

For example compounds having formula 1 wherein $R_5$ is hydrogen and Z is methylene optionally substituted with alkyl having 1-3 C-atoms can be obtained by reacting a compound of the formula 2

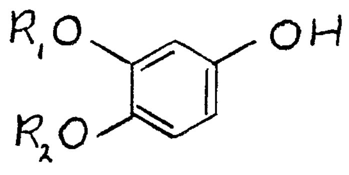

(2)

with a compound of the formula $R_3R_4NH$ and an aldehyde of the formula $R_7CHO$, in which formulae $R_1$-$R_4$ have the above mentioned meaning and $R_7$ is hydrogen or alkyl having 1-3 C-atoms. This so-called Mannich-reaction is preferably carried out in an inert organic solvent.

The starting compounds of formula 2 are known or can be obtained analogously to known compounds.

The so-obtained compounds of formula 1 wherein $R_5$ is hydrogen can be converted into the corresponding compounds wherein $R_5$ has one of the other meanings by means of a reaction with a compound of the formula $R'_5$-X, wherein $R'_5$ has the same meaning as $R_5$ except hydrogen, and X is a so-called leaving group, for example halogen. This reaction is preferably carried out in an inert solvent such as dimethylsulphoxide (DMSO), N,N-dimethylformamide (DMF) and the like, in the presence of a suitable base such as sodium hydride or potassium tert-butoxide and the like. Sometimes the addition of sodium iodide is desirable. The reaction may be carried out at somewhat elevated temperatures.

Compounds of the formula 1 wherein Z is methylene optionally substituted with alkyl having 1-3 C-atoms, and $R_4$ is hydrogen can be obtained by the reductive amination reaction of a compound of formula 3

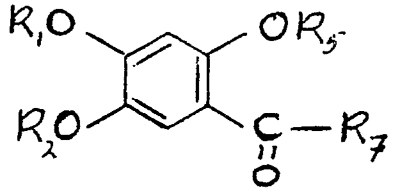

(3)

with a primary amine of the formula $R_3NH_2$, in which formulae $R_1$-$R_3$ and $R_5$ have the meanings given in formula 1, and $R_7$ is hydrogen or alkyl having 1-3 C-atoms. Addition of an acid catalyst may be desirable to

enhance the reaction rate. The reaction is preferably carried out in an inert solvent. Removal of water formed during the reaction is preferably carried out during the amination step by means of azeotropic distillation with an aromatic solvent, for example benzene or toluene. The reductive step of this process can be carried out with a suitable reducing agent such as $LiAlH_4$ in an inert solvent, for example tetrahydrofuran or diethyl ether.

Starting compounds of the formula 3 are known (J.Chem.Soc. Perkin I(1974), 305; Synth.Commun. 10 , (1980), 9), or can be prepared in a similar manner.

The so-obtained compounds of formula 1, wherein $R_4$ is hydrogen can be converted into the corresponding compounds wherein $R_4$ has another meaning by reductive alkylation with a suitable aldehyde in the presence of a reducing agent such as sodium cyanoborohydride in an inert solvent.

The above described reductive amination of a compound of formula 3 can also be carried out with a secondary amine of the formula $R_3R_4NH$, leading directly to the desired end products of formula 1.

Compounds of formula 1 wherein Z is ethylene, optionally substituted with one alkyl group having 1-3 C-atoms, can be prepared according to known methods for the preparation of phenethylamines (Roth, Kleemann, "Pharmaceutical Chemistry", vol.1, pages 38-69, John Wiley and Sons, New York 1988)

The invention will be further illustrated by means of the following examples.

## Example I

### 5-N,N-diethylaminomethyl-6-hydroxy-1,3-benzodioxole

To a stirred solution of 5-hydroxy-1,3-benzodioxole (5,0 g, 36,2 mmol) and diethylamine (2,9 g, 39,7 mmol) in ethanol (25 ml) was added dropwise 37% aqueous formaldehyde (3,2 g, 39,5 mmol). The reaction mixture was stirred at room temperature for 4 hours and evaporated in vacuo. The remaining oil (7,4 g) was crystallized from methanol; melting point 52-54 °C (compound no. 1).
In a similar manner the compounds of formula 1 wherein $R_5$ is hydrogen, Z is methylene and $R_1$-$R_4$ have the meaning indicated in table A were prepared

Table A

| Compound No. | $R_1$ (+) $R_2$ | $R_3$ (+) $R_4$ | m.p.(°C) |
|---|---|---|---|
| 2 | $-(CH_2)-$ | $-(CH_2)_2O(CH_2)_2-$ | 86- 88 |
| 3 | $-(CH_2)-$ | $-(CH_2)_5-$ | 50- 51 |
| 4 | $-(CH_2)-$ | $-(CH_2)_2N(CH_3)(CH_2)_2-$ | 95- 97 |
| 5 | $-(CH_2)_2-$ | $C_2H_5$ $C_2H_5$ | oil |
| 6 | $-(CH_2)_2-$ | $-(CH_2)_5-$ | oil |
| 7 | $CH_3$ $CH_3$ | $C_2H_5$ $C_2H_5$ | oil |
| Compound No. | $R_1$ (+) $R_2$ | $R_3$ (+) $R_4$ | m.p.(°C) |
| 8 | $CH_3$ $CH_3$ | $-(CH_2)_5-$ | 38- 39 |
| 9 | $-(CH_2)-$ | $-(CH_2)_2N(CH(C_6H_5)_2)(CH_2)_2-$ | 160-161 |

## Example II

### 5-N,N-diethylaminomethyl-6-phenylmethoxy-1,3-benzodioxole.HCl

To a mixture of 5-N,N-diethylaminomethyl-6-hydroxy-1,3-benzodioxole (2.0 g, 9 mmol), sodium iodide (0.135 g, 0.9 mmol) and potassium-tert-butoxide (1,19 g, 10.5 mmol) in DMSO (30 ml) was added benzylbromide (1.54 g, 9 mmol). The stirred mixture was heated at 80 °C for 3 hours. Thereafter, the

mixture was allowed to attain room temperature, water was added and the resulting solution was extracted with dichloromethane, washed with a 2N sodium hydroxide solution and brine respectively, dried over $MgSO_4$, filtered and evaporated in vacuo. The obtained brown oil (3.7 g) was purified by flash chromatography (silicagel, dichloromethane/methanol/ammonia(25%) = 95/4.5/0.5) to give pure 5-N,N-diethylaminomethyl-6-phenylmethoxy-1,3-benzodioxole (2.0 g, yield 71%). A mixture of isopropanol and diethyl ether was added to the obtained product, and the resulting solution was saturated with gaseous hydrogen chloride. After evaporation of the solvents in vacuo 5-N,N-diethylaminomethyl-6-phenylmethoxy-1,3-benzodioxole.HCl (1.4 g, yield 45%) was obtained as a white solid; m.p. l22-123 °C (compound no. 10).

In a similar manner the compounds of formula 1 wherein Z is methylene and $R_1$-$R_5$ have the meaning indicated in table B were prepared:

Table B

| No | R1 (+) R2 | R3 | (+) | R4 | R5 | salt | m.p.(°C) |
|---|---|---|---|---|---|---|---|
| 11 | $-(CH_2)-$ | $CH_3$ | | $CH_3$ | $n-C_3H_7$ | $HCl.0.1\ H_2O$ | 138-140 |
| 12 | $-(CH_2)-$ | $CH_3$ | | $CH_3$ | $n-C_4H_9$ | HCl | 140-142 |
| 13 | $-(CH_2)-$ | $CH_3$ | | $CH_3$ | $n-C_5H_{11}$ | HCl | 131-133 |
| 14 | $-(CH_2)-$ | $CH_3$ | | $CH_3$ | $n-C_6H_{13}$ | citric acid | 119-121 |
| 15 | $-(CH_2)-$ | $CH_3$ | | $CH_3$ | $n-C_8H_{17}$ | HCl | 113-115 |
| 16 | $-(CH_2)-$ | $CH_3$ | | $CH_3$ | $n-C_{12}H_{25}$ | HCl | 120-122 |
| 17 | $-(CH_2)-$ | $CH_3$ | | $CH_3$ | $(CH_2)_3C_6H_5$ | HCl | 167-169 |
| 18 | $-(CH_2)-$ | $C_2H_5$ | | $C_2H_5$ | $n-C_3H_7$ | $HCl.0.1\ H_2O$ | 111-113 |
| 19 | $-(CH_2)-$ | $C_2H_5$ | | $C_2H_5$ | $n-C_4H_9$ | $HCl.0.1\ H_2O$ | 122-124 |
| 20 | $-(CH_2)-$ | $C_2H_5$ | | $C_2H_5$ | $n-C_5H_{11}$ | HCl | 120-122 |
| 21 | $-(CH_2)-$ | $C_2H_5$ | | $C_2H_5$ | $n-C_6H_{13}$ | HCl | 127-129 |
| 22 | $-(CH_2)-$ | $C_2H_5$ | | $C_2H_5$ | $n-C_8H_{17}$ | HCl | 131-132 |
| 23 | $-(CH_2)-$ | $C_2H_5$ | | $C_2H_5$ | $n-C_{12}H_{25}$ | HCl | 110-112 |
| 24 | $-(CH_2)-$ | $C_2H_5$ | | $C_2H_5$ | $(CH_2)_3C_6H_5$ | HCl | 115-117 |
| 25 | $-(CH_2)-$ | $n-C_3H_7$ | | $n-C_3H_7$ | $n-C_5H_{11}$ | HCl | 111-113 |
| 26 | $-(CH_2)-$ | $i-C_3H_7$ | | $i-C_3H_7$ | $n-C_5H_{11}$ | HCl | 117-119 |
| 27 | $-(CH_2)-$ | $n-C_4H_9$ | | $n-C_4H_9$ | $n-C_5H_{11}$ | HCl | 114-116 |
| 28 | $-(CH_2)-$ | $n-C_5H_{11}$ | | $n-C_5H_{11}$ | $n-C_5H_{11}$ | oxalic acid | 124-126 |
| 29 | $-(CH_2)-$ | $n-C_6H_{13}$ | | $n-C_6H_{13}$ | $n-C_5H_{11}$ | oxalic acid | 100-102 |
| 30 | $-(CH_2)-$ | cyclohexyl | | cyclohexyl | $n-C_5H_{11}$ | HCl | 183-185 |

| No | R1 (+) R2 | R3 | (+) | R4 | R5 | salt | m.p. (°C) |
|---|---|---|---|---|---|---|---|
| 31 | $-(CH_2)-$ | $C_2H_5$ | | $C_2H_5$ | $CH_2C(O)C_6H_5$ | fumaric acid | 161-163 |
| 32 | $-(CH_2)-$ | | $-(CH_2)_5-$ | | $n-C_4H_9$ | HCl | 162-164 |
| 33 | $-(CH_2)-$ | | $-(CH_2)_5-$ | | $n-C_5H_{11}$ | HCl | 151-153 |
| 34 | $-(CH_2)-$ | | $-(CH_2)_5-$ | | $(CH_2)_2CH(CH_3)_2$ | HCl | 192-194 |
| 35 | $-(CH_2)-$ | | $-(CH_2)_5-$ | | $CH_2C_6H_5$ | free base | 73- 75 |
| 36 | $-(CH_2)-$ | | $-(CH_2)_5-$ | | $CH_2-2-Cl-C_6H_4$ | free base | 82- 84 |
| 37 | $-(CH_2)-$ | | $-(CH_2)_5-$ | | $CH_2-3-Cl-C_6H_4$ | HCl | 213-216 |
| 38 | $-(CH_2)-$ | | $-(CH_2)_5-$ | | $CH_2-4-Cl-C_6H_4$ | HCl | 207-209 |
| 39 | $-(CH_2)-$ | | $-(CH_2)_5-$ | | $CH_2-2-F-C_6H_4$ | free base | 101-103 |
| 40 | $-(CH_2)-$ | | $-(CH_2)_5-$ | | $CH_2-3-F-C_6H_4$ | HCl.½$CH_3OH$ | 180-182 |
| 41 | $-(CH_2)-$ | | $-(CH_2)_5-$ | | $CH_2-4-F-C_6H_4$ | HCl.½$CH_3OH$ | 211-213 |
| 42 | $-(CH_2)-$ | | 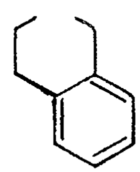 | | $n-C_5H_{11}$ | HCl | 157-159 |
| 43 | $-(CH_2)-$ | | $-(CH_2)_4-$ | | $n-C_5H_{11}$ | HCl | 156-158 |
| 44 | $-(CH_2)-$ | $-(CH_2)_2O(CH_2)_2-$ | | | $n-C_4H_9$ | HCl | 212-215 |
| 45 | $-(CH_2)-$ | $-(CH_2)_2N(CH_3)(CH_2)_2-$ | | | $(CH_2)_3C_6H_5$ | 2HCl | 245(dec) |
| 46 | $-(CH_2)-$ | $-(CH_2)_2N(CH_3)(CH_2)_2-$ | | | $n-C_4H_9$ | HCl.2$H_2O$ | 197-200 |
| 47 | $-(CH_2)-$ | $-(CH_2)_2N(CH_3)(CH_2)_2-$ | | | trans-$CH_2CHCHC_6H_5$ | free base | 225-227 |
| 48 | $-(CH_2)-$ | $-(CH_2)_2N(CH(C_6H_5)_2)(CH_2)_2-$ | | | $CH_3$ | 2HCl | 222-224 |

| No | R1 | (+) | R2 | R3 | (+) | R4 | R5 | salt | m.p. (°C) |
|---|---|---|---|---|---|---|---|---|---|
| 49 | | $-(CH_2)-$ | | | $-(CH_2)_2N(CH(C_6H_5)_2)(CH_2)_2-$ | | $n-C_4H_9$ | 2HCl | 230-231 |
| 50 | | $-(CH_2)-$ | | | $-(CH_2)_2N(CH_2-3,4-OCH_2O-C_6H_3)(CH_2)_2-$ | | $n-C_4H_9$ | 2HCl | 257-260 |
| 51 | | $-(CH_2)_2-$ | | $C_2H_5$ | | $C_2H_5$ | $(CH_2)_3C_6H_5$ | fumaric acid | 131-133 |
| 52 | | $-(CH_2)_2-$ | | $C_2H_5$ | | $C_2H_5$ | $n-C_5H_{11}$ | fumaric acid | 107-109 |
| 53 | | $-(CH_2)_2-$ | | | $-(CH_2)_5-$ | | $(CH_2)_3C_6H_5$ | HCl | 168-170 |
| 54 | | $-(CH_2)_2-$ | | | $-(CH_2)_5-$ | | $n-C_5H_{11}$ | HCl | 169-171 |
| 55 | $CH_3$ | | $CH_3$ | $C_2H_5$ | | $C_2H_5$ | $(CH_2)_3C_6H_5$ | HCl | 138-140 |
| 56 | $CH_3$ | | $CH_3$ | $C_2H_5$ | | $C_2H_5$ | $n-C_5H_{11}$ | HCl | 128-129 |
| 57 | $CH_3$ | | $CH_3$ | | $-(CH_2)_5-$ | | $(CH_2)_3C_6H_5$ | HCl | 142-143 |
| 58 | $CH_3$ | | $CH_3$ | | $-(CH_2)_5-$ | | $n-C_5H_{11}$ | fumaric acid | 138-140 |

Table C

| No | R1 + R2 | R3 | R4 | R5 | salt | m.p.(°C) |
|---|---|---|---|---|---|---|
| 60 | $-(CH_2)-$ | $CH_3$ | $CH_3$ | $C(O)-n-C_3H_7$ | HCl | 184-186 |
| 61 | $-(CH_2)-$ | $CH_3$ | $CH_3$ | $C(O)-n-C_4H_9$ | HCl | 154-158 |
| 62 | $-(CH_2)-$ | $C_2H_5$ | $C_2H_5$ | $C(O)-n-C_4H_9$ | HCl | 123-125 |

Table D

| No | R1 + R2 | R3 | R4 | R5 | salt | m.p.(°C) |
|---|---|---|---|---|---|---|
| 64 | $-(CH_2)-$ | H | $(CH_2)_2$-3,4-$(OCH_3)_2$-$C_6H_3$ | n-$C_5H_{11}$ | HCl | 143-145 |
| 65 | $-(CH_2)-$ | H | $(CH_2)_2$-3,4-$(OCH_3)_2$-$C_6H_3$ | $(CH_2)_3C_6H_5$ | HCl | 117-119 |
| 66 | $-(CH_2)-$ | H | $(CH_2)_2$-3,4-$(OCH_3)_2$-$C_6H_3$ | $CH_2C_6H_5$ | HCl | 185-188 |
| 67 | $-(CH_2)-$ | H | $(CH_2)_2$-3,4-$(OCH_3)_2$-$C_6H_3$ | $(CH_2)_5C_6H_5$ | HCl | 138-140 |
| 68 | $-(CH_2)-$ | H | $(CH_2)_2$-3,4-$OCH_2O$-$C_6H_3$ | $CH_2C_6H_5$ | HCl | 188-190 |

Example III

1,3-benzodioxole-5-N,N-diethylaminomethyl-6-n-butyrate.HCl.

A mixture of 5-N,N-diethylaminomethyl-6-hydroxy-1,3-benzodioxole (3.0 g, 13.5 mmol), n-butyric anhydride (2.15 g, 13.6 mmol) and a drop of concentrated sulphuric acid was stirred at room temperature for 15 minutes. Crushed ice was added to the clear solution. The pH of the solution was made alkaline (pH = 10-11) by means of a 10% NaOH solution. The mixture was extracted with diethyl ether and the combined ether extract was washed with water, dried over $Na_2SO_4$ and evaporated under reduced pressure. The resulting oil was dissolved in diethyl ether and saturated with gaseous hydrochloric acid. The precipitated 1,3-benzodioxole-5-N,N-diethylaminomethyl-6-n-butyrate.HCl (compound no. 59) was filtered off and recrystallized from methanol/diethyl ether; m.p. 126-128 °C.

In a similar manner the compounds having formula 1 wherein Z is methylene and $R_1$-$R_5$ have the meaning mentioned in table C were obtained.

Example IV

5-N-(2-phenylethyl)aminomethyl-6-phenylmethoxy-1,3-benzodioxole.HCl.

To a solution of 1,3-benzodioxole-5-phenylmethoxy-6-carboxaldehyde (2.0 g, 7.8 mmol) in benzene (75 ml) were added phenethylamine (0.94 g, 7.8 mmol) and a catalytic amount of p-toluenesulphonic acid. The reaction mixture was heated at reflux temperature overnight using a Dean-Stark trap. The reaction mixture was cooled to room temperature and evaporated in vacuo. The residue was dissolved in dry THF, and lithiumaluminium hydride (0.6 g, 16 mmol) was added. The mixture was heated at reflux temperature for 20 hours under nitrogen and then cooled to room temperature. Excess $LiAlH_4$ was decomposed by cautious addition of $Na_2SO_4.10\ H_2O$. The solid was filtered off and washed with two portions of hot tetrahydrofuran. The combined THF solution was evaporated under reduced pressure to give a yellow oil. The oil was dissolved in isopropanol/diethyl ether and saturated with gaseous hydrochloride. The precipitated 5-N-(2-phenylethyl)aminomethyl-6-phenylmethoxy-1,3-benzodioxole.HCl (compound no. 63) was filtered off and recrystallized from ethyl acetate; melting point 188-190 °C.

In a similar manner compounds of formula 1 wherein Z is methylene and $R_1$-$R_5$ have the meaning given in table D were obtained.

Example V

5-N-[2-(3,4-dimethoxyphenyl)ethyl]-N-methyl-aminomethyl-6-phenylmethoxy-1,3-benzodioxole.

Sodium cyanoborohydride (0.34 g, 5.4 mmol) was added to a solution of 5-N-[2-(3,4-dimethoxyphenyl)-ethyl]aminomethyl-6-phenylmethoxy-1,3-benzodioxole (1.42 g, 3.37 mmol) and aqueous formaldehyde (37% solution, 1.25 ml, 15.4 mmol) in acetonitrile (10 ml). The reaction mixture was stirred for 20 minutes at room temperature. The reaction mixture was made neutral by dropwise addition of glacial acetic acid. Stirring was continued for 5 hours. The solvent was removed under reduced pressure and the residue was partitioned between 2N potassium hydroxide solution (25 ml) and diethyl ether (25 ml). The aqueous layer was further extracted with two portions of diethyl/ether and the combined organic extract was washed with 0.5N KOH solution and with water. The ether solution was extracted with 10% hydrogen chloride solution. The combined aqueous extract was made alkaline with sodium hydroxide pellets and the solution was extracted with diethyl ether. The combined diethyl ether extract was washed with water, dried over $Na_2SO_4$ and evaporated under reduced pressure to give 1.03 g (yield 70%) of 5-N-[2-(3,4-dimethoxyphenyl)ethyl]-N-methyl-aminomethyl-6-phenylmethoxy-1,3-benzodioxole (compound no. 69); melting point 78-80 °C.

## Claims

1. Compositions having anti-ischaemic activity which comprise as an active component at least one compound of the formula

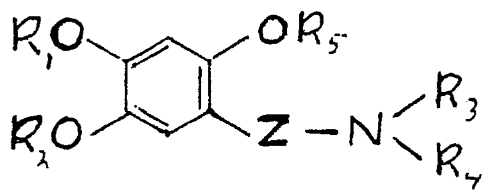

(1)

wherein:
-$R_1$ and $R_2$ independent of each other represent hydrogen, alkyl having 1-3 C-atoms, on the understanding that $R_1$ and $R_2$ are not both hydrogen, or wherein $R_1 + R_2$ together form an alkylene group having 1-3 C-atoms which may be substituted with one or more alkyl groups having 1-3 C-atoms;
-Z is methylene optionally substituted with one alkyl group having 1-3 C-atoms, or Z is ethylene of which one or both carbon atoms may be substituted with one alkyl group having 1-3 C-atoms;
-$R_3$ and $R_4$ independent of each other represent hydrogen, alkyl having 1-10 C-atoms, alkenyl or alkynyl having 3-10 C-atoms, cycloalkyl having 3-8 C-atoms, cycloalkyl-alkyl having 3-8 ring atoms and 1-5 C-atoms in the alkyl group, phenylalkyl or heteroaryl-alkyl having 1-5 C-atoms in the alkyl group, phenylalkenyl, heterary-alkenyl, phenylalkynyl or heteroaryl-alkynyl group having 3-5 C-atoms in the alkenyl group or alkynyl group, which groups $R_3$ and R4 may be substituted with a group $(R_6)_p$ wherein $R_6$ is halogen, hydroxy, alkyl or hydroxyalkyl having 1-5 C-atoms, alkoxy having 1-3 C-atoms, S-alkyl, S(O)-alkyl or $S(O)_2$-alkyl having 1-3 C-atoms, amino, mono- or dialkylamino having 1-3 C-atoms per alkyl group, trifluoromethyl, trifluoromethoxy or cyano, and p has the value 0-3, on the understanding that $R_3$ and $R_4$ are not both hydrogen, or wherein $R_3 + R_4$ together with the nitrogen atom form a saturated or unsaturated heterocyclic group of 5 or 6 ringatoms, which may contain a second hetero-atom from the group consisting of oxygen, sulphur and nitrogen, which ring may be substituted with phenylalkyl having 1-3 C-atoms in the alkyl group, or with a group $(R_6)_p$ wherein $R_6$ and p have the above-mentioned meanings, or which ring may be annelated with a phenyl group;
-$R_5$ is hydrogen, alkyl having 1-12 C-atoms, alkenyl or alkynyl having 3-12 C-atoms, cycloalkyl having 3-8 C-atoms, cycloalkyl-alkyl having 3-8 ring atoms and 1-5 C-atoms in the alkyl group, phenylalkyl or heteroaryl-alkyl having 1-5 C-atoms in the alkyl sub-group, phenylalkenyl, heteroaryl-alkenyl, phenylalkynyl or heteroaryl-alkynyl having 3-5 C-atoms in the alkenyl sub-group or alkynyl sub-group, which groups may be substituted with a group $(R_6)_p$, wherein R6 and p have the above-mentioned meaning, and which alkyl sub-groups, alkenyl sub-groups and alkynyl sub-groups may contain a group -O-, -S- or CO, or a pharmacologically acceptable salt thereof.

2. A method of preparing compositions as claimed in claim 1, characterized in that a compound of formula 1 wherein the symbols have the meaning mentioned in claim 1, or a salt thereof, is brought into a form suitable for administration.

3. Compounds of formula 1, wherein the symbols have the meaning given in claim 1, or a salt thereof, with the exception of the compound wherein $R_1$ + $R_2$ form a methylene group, $R_3$ = $R_4$ = $CH_3$, $R_5$ is hydrogen, and Z is the methylene group.

4. A method of preparing phenylalkyl amine derivatives, characterised in that compounds having formula 1, wherein Z is methylene which may be substituted with alkyl having 1-3 C-atoms, and $R_1$-$R_5$ have the meanings given in claim 1 are prepared by reacting a compound having formula 2

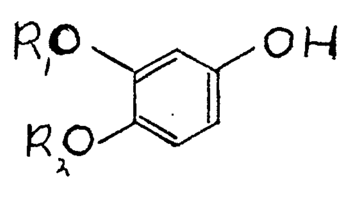

with a compound of the formula $R_3R_4NH$ and an aldehyde of the formula $R_7CHO$, wherein $R_7$ is hydrogen or alkyl having 1-3 C-atoms, and optionally converting the obtained compound wherein $R_5$ is hydrogen, with

a compound of the formula $R_5'$-X, wherein $R_5'$ has the same meaning as $R_5$ with the exception of hydrogen.

5. A method as claimed in claim 4, characterized in that compounds having formula 1 wherein Z is methylene which may be substituted with alkyl having 1-3 C-atoms and $R_1$-$R_4$ have the meanings given in claim 1, are prepared by reacting a compound of formula 3

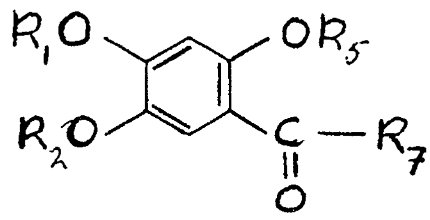

wherein $R_7$ is hydrogen or alkyl with 1-3 C-atoms, with a primary amine of the formula $R_3NH_2$ and optionally converting the obtained compound wherein $R_4$ is hydrogen into a compound wherein $R_4$ has one of the other meanings given in claim 1 by means of reductive alkylation.

6. A method as claimed in claim 4, characterized in that compounds having formula 1 wherein Z is ethylene which may be substituted with one alkyl group having 1-3 C-atoms, and $R_1$-$R_5$ have the meanings given in claim 1, are prepared according to methods suitable for the preparation of phenethylamines.

7. A method of treating ischaemia, characterized in that a composition according to claim 1 is used.

European Patent Office

**PARTIAL EUROPEAN SEARCH REPORT**
which under Rule 45 of the European Patent Convention shall be considered, for the purposes of subsequent proceedings, as the European search report

Application number
EP 89 20 2383

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 106, 1987, page 16, abstract no. 60775y, Columbus, Ohio, US; J.K. BATRA et al.: "Morpholino derivatives of benzylbenzodioxole. A study of structural requirements for drug interactions at the colchicine/podophyllotoxin binding site of tubulin" & BIOCHEM. PHARMACOL. 1986, 35(22), 4013-18<br>* Abstract * | 1,2 | C 07 D 317/64<br>C 07 C 217/58<br>A 61 K 31/36 |
| | -- | | |
| A | FR-A-2 258 176 (HOECHST)<br>* Pages 1-3,8; claims * | 1,4,5 | |
| | ---- | | |

TECHNICAL FIELDS SEARCHED (Int. Cl.4)

C 07 D 317/00
C 07 C 93/00

**INCOMPLETE SEARCH**

The Search Division considers that the present European patent application does not comply with the provisions of the European Patent Convention to such an extent that it is not possible to carry out a meaningful search into the state of the art on the basis of some of the claims.

Claims searched completely: 1-6
Claims searched incompletely:
Claims not searched: 7
Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 11-05-1990 | FRANÇOIS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82